(19) **Europäisches Patentamt**
**European Patent Office**
**Office européen des brevets**

(11) **EP 1 606 381 B1**

(12) **EUROPEAN PATENT SPECIFICATION**

(45) Date of publication and mention
of the grant of the patent:
**27.05.2009 Bulletin 2009/22**

(51) Int Cl.:
*C11D 17/00* (2006.01)  *C12N 9/98* (2006.01)
*C11D 3/386* (2006.01)  *A23K 1/00* (2006.01)

(21) Application number: **04720589.3**

(22) Date of filing: **15.03.2004**

(86) International application number:
**PCT/DK2004/000167**

(87) International publication number:
**WO 2004/082564 (30.09.2004 Gazette 2004/40)**

(54) **COATED ENZYME GRANULES**

UMHÜLLTE ENZYMKÖRNCHEN

GRANULES ENZYMATIQUES REVETUS

(84) Designated Contracting States:
**AT BE BG CH CY CZ DE DK EE ES FI FR GB GR
HU IE IT LI LU MC NL PL PT RO SE SI SK TR**

(30) Priority: **18.03.2003 DK 200300412**

(43) Date of publication of application:
**21.12.2005 Bulletin 2005/51**

(73) Proprietor: **Novozymes A/S**
**2880 Bagsvaerd (DK)**

(72) Inventor: **SIMONSEN, Ole**
**Søborg 2860 (DK)**

(56) References cited:
**EP-A- 0 256 127**     **WO-A-01/23513**
**WO-A-02/28369**     **WO-A-96/16151**
**US-A- 4 078 099**     **US-B1- 6 245 384**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to granules comprising a core and a coating wherein the core comprises an active compound which is an enzyme and the coating comprises a wax composition with a wide molecular weight distribution which improves the protection against the release of active compound dust from the granule, The present invention further relates to processes for the manufacture of such coated granules.

**BACKGROUND OF THE INVENTION**

It is known to the art to incorporate active compounds such as enzymes into dry solid particles or granules and thereby protect the active compounds from inactivation and/or protect the environment from the active compounds. Such particles or granules have usually been applied to other dry products such as dry granular detergent compositions to improve their performance. Enzymes are examples of active compounds, which may be incorporated in dry solid particles or granules.

Reasons for formulating active compounds into particles, such as preparing enzyme granules include protection of the active compound by separating it from the surrounding potentially hostile environment until the moment when the active compound is to be used in an application and reduction of potentially harmful dust which may be generated from the active compound upon handling of the enzymes. Said protection: of the active compound and reduction of dust formation may, in accordance with prior art be aided or improved by coating the particles.

It is known to the art to prepare granules comprising an active-containing core and various coating layers. A granule comprising active compounds typically consist of a core and a coating, where an important feature of the coating is to reduce the formation of dust when handling the granulate.

A T-granulate coated with a wax, a triglyceride or other fat is described in WO. 89/08694

WO 96/16151 relates to enzyme granules coated with an unctuous coating of a non aqueous liquid having dissolved a component of a melting point of 30-90°C.

US 5,480,577 describes parties coated with a water insoluble paraffin was having a melting point between 40°C to 50°C, and a solids content of 0% to 15% at 50°C, the paraffin optionally combined with 70% to 1% of polyvinyl ether.

EP-A-256 127 discloses enzyme granules having a coating of polyethylene glycol wax.

**SUMMARY OF THE INVENTION**

One object of the present invention is to provide coated granules comprising enzymes wherein the coating provides acceptable low release of active dust from the granules upon handling. A second object of the present invention is to provide coated granules enzymes wherein the coating provides adequate protection of the enzymes in the granules against hostile compounds in the surrounding environment. A third object of the invention is to provide coated granules comprising enzymes wherein the coating provides adequate protection against rough handling.

We have surprisingly found that coating of particles, comprising enzymes with a wax composition comprising a wide or broad molecular weight distribution improves Heubach dust figures of the coated finished granules significantly compared to ordinary granules typically coated with a wax composition comprising a narrow molecular weight distribution. We have further found that by using a wax composition comprising a wide molecular weight distribution in the coating, the coating becomes more elastic compared to granules coated with a wax composition comprising a narrow molecular weight distribution.

Hence the present invention provides a granule comprising a core and a coating wherein the core comprises an active compound which is an enzyme and the coating comprises a wax composition with a molecular weight distribution of:

(a) at least 10% w/w in the range 0.25xMw to 0.75×Mw, and
(b) at least 20% w/w in the range 0.75Mw to 1.25×Mw, and
(c) at least 10% w/w in the range 1.25×Mw to 2.0×Mw,

where Mw is the weight average molecular weight of the wax composition,

The invention further provides methods for preparation for preparing the coated granules of the invention, and uses of said granules.

## DETAILED DESCRIPTION OF THE INVENTION

### Definitions

The average molecular weight "Mw" in the context of the invention is for a wax composition and is defined as:

$$Mw = \sum_{i=1}^{\infty} w_i \times M_i$$

where $w_i$ is the weight fraction of each molecular weight $M_i$ present in the wax composition.

By the term "a wide molecular weight distribution" when used to characterize a wax composition it is meant that the wax composition comprises polymers for which there are great variations in the molecular weights of the specific polymers. The range of molecular weights of individual polymers are thus so broad so than at least 10% w/w of the molecular weig ht is in the range of 0.25 x Mw to 0.75 x Mw, and at least 20% w/w of the molecular weight is in the range 0.75x Mw to 1.25 x Mw and at least 10% w/w of the molecular weight is in the range 1.25 x Mw to 2.0 x Mw, where Mw is the average molecular weight of the wax composition.

By the term "narrow molecular weight distribution" when used about a wax composition it is meant that the wax compositions have a narrower molecular weight distribution than defined above for a "wide molecular weight distribution". In particular the molecular weights of polymers in the wax composition follow approximately a Poisson distribution. The term approximately is used herein as an indication that the distribution of a real life composition in most cases is not perfectly Poisson distributed. It may be a wax composition with a specified average molecular weight informed by the supplier when obtaining the wax composition commercially e.g. PEG 4000.

The terms "particles" and "granules" in the context of the present invention are to be understood as a predominantly spherical or near spherical structure of a macromolecular size and coated particles are in the following referred to as granules.

The term "caking strength" in the context of the invention is in principle the force required to break granules apart after removal of a pressure applied to granules, which have caused the granules to stick together. The "caking strength" is also called the unconfined yield strength.

The "caking strength" of the present invention is measured by the following method: A container (a cylinder with an inner diameter of 64 mm, and height of 152 mm) is placed on a horizontal plate and filled to the top with granulate. A 5 kg weight fitting into the container is placed on top of the granules and removed after two minutes together with the container; if the cake of granules collapses the caking strength is zero. Weights of 100 g are placed on top of the free standing cake of granules and on top of each other until the cake collapses. The caking strength is the sum of the weights minus 50 g.

A Poisson distributed polymer is where the fraction of molecules with *n* repeating units is given by

$$F(n) = e^{-m} \left[ \frac{m^n}{n!} \right]$$

where *m* is the average number of repeating units, and n and m are integers.

The term "substantially free of active compound" as used herein about a coating means that the coating contains less than 5 mg of active compound per gram coating. In practice no active compound is added to the coating feed.

### Introduction

When handling solid particles comprising an enzyme, one of the major problems is the formation of dust from the active compound, which may be harmful to persons handling the enzymes.

Although the enzyme may be incorporated in dry solid granules as known to the art, which may inhibit the formation of active dust it is a fact that conventional granules are usually solid and brittle which makes them susceptible to damage when a strain is applied to them, which might happen during handling such as manufacture, packaging and transportation.

It is presently contemplated that dust is released when the integrity of solid particles is damaged, e.g. when a solid particle and/or its coating breaks or cracks. This may happen when the solid particle is subjected to strain such as impact or shear e.g. during handling. The strain will cause a corresponding stress building up in the solid particle to counteract the strain. Upon increasing the strain the build up of stress in the particle to counteract the strain may continue to a certain point (the yield point) depending on the particle material. However, if the strain is greater than the forces upholding the integrity of the solid particle (the yield point) the solid particle is no longer able of counteracting the strain and the strain will cause damage to the physical integrity of the solid particle, which may release dust from the solid particle. This may happen when the particle is exposed to shear or impact, especially particles with high surface roughness cause friction and the edges and corners on the surface of the particle will act as weak areas which are susceptible to breaking off as fragments of the particle or to induce breakage of the entire particle.

By coating granules comprising an enzyme with a coating layer comprising a wax composition comprising a wide molecular weight distribution, the inventors have surprisingly found, it is possible to obtain much lower dust figures for the granules compared to granules coated with a wax composition comprising a narrow molecular weight distribution. It is furthermore found that by using a wax composition comprising a wide molecular weight distribution the surface of the coating becomes more smooth, less crystalline and less brittle and as a result the coating of the invention is not as susceptible to forming cracks as known coatings are, and hence the stability of the granules is improved towards hostile components in the near environment of the granules, as the hostile components can not get in contact with the enzyme through cracks in the coating.

If two or more wax compositions, comprising narrow molecular weight distributions, are mixed together to obtain a wax composition comprising a wide molecular weight distribution, it is preferred that the different wax compositions comprises molecular weights which overlap. It is more preferred that the wax composition comprising a wide molecular weight distribution in the context of the invention is an approximately uni-modal molecular weight distribution wax composition.

One way of obtaining such a wax composition comprising a wide molecular weight distribution suitable in the present invention is by mixing two or more different wax compositions comprising narrow molecular weight distributions. The mixing of different wax compositions comprising narrow molecular weight distributions may be done by the supplier or by the granulate manufacturer. Another way of obtaining a wax composition comprising a wide molecular weight distribution is from a manufacturer of synthetic waxes, which has been able of producing a wax composition comprising a wide molecular weight distribution during the polymerisation process or from natural sources if naturally occurring waxes can be found with a wide molecular weight distribution as defined vide supra.

When using a wax composition comprising a narrow molecular weight distribution as coating material, a crystalline structured coating is obtained and the crystalline coating is susceptible to cracking. By using a wax composition comprising a wide molecular weight distribution as a coating material, the inventors have found that a more amorphous coating is obtained which is not as susceptible to cracking.

When using a wax composition of low molecular weight e.g. PEG 400 the coating may become unctuous, due to the low molecular weight wax compositions are viscous liquids at room temperature and the granules will become sticky and unmanageable. The caking strength can tell something about how sticky the granules are. The higher the caking strength the stickier the granules are. To be able to handle the granules of the present invention the caking strength is preferably below 1000, more preferably below 800.

The inventors have further observed that when coating with a mixture of a high molecular weight wax composition (e.g. PEG 4000) and a low molecular weight wax composition (e.g. PEG 400) the coating becomes sticky and the granules become unmanageable.

Most synthetic polymer waxes are produced with a narrow molecular weight distribution. Some synthetic polymer waxes like polyethylene glycol (PEG, $H(OCH_2CH_2)_nOH$) and alkylphenol ethoxylates ($RO(CH_2CH_2O)_n$ where R is an alkylphenol) are produced with a molecular weight distribution following a Poisson distribution (around the number of repeating units n), giving a very narrow molecular weight distribution for high n-values. Other synthetic waxes like fatty alcohol ethoxylates ($RO(CH_2CH_2O)_n$ where R is a fatty alcohol) follow other distributions e.g. Weibull-Nycander-Gold type distributions depending on the polymeric reaction, choice of catalyst etc., but still typically resulting in a relatively narrow distribution around a mean molecular weight. References see "Chemical Fact Sheet: Nonionic Surfactants" from Orica, Sallay, P. et al, "Molar Mass Distribution of Hydroxyethylated Aralkyl Alcohols", Periodica Polytechnica Ser. Chem. Eng. Vol. 44, No. 2, pp. 95-110 (2000) and "Polyethylene Glycol, Product information" from Clariant

## The coating

The wax coating of the present invention comprises a wax composition comprising a wide molecular weight distribution, that is at least 10% w/w of the waxes have a molecular weight of 0.25xMw to 0.75xMw, and at least 20% w/w of the waxes have a molecular weight of 0.75xMw to 1.25xMw, and at least 10% w/w of the waxes have a molecular weight of 1.25xMw to 2.0xMw, where Mw is the weight average molecular weight of the wax composition.

In particular the amount of waxes having a molecular weight of 0.25xMw to 0.75xMw is at least 15% w/w, e.g. at least 18% w/w, e.g. at least 20% w/w, e.g. at least 22% w/w, e.g. at least 24% w/w, e.g. at least 26% w/w, e.g. at least 28% w/w, e.g. at least 30% w/w, e.g. at least 35% w/w, e.g. at least 40% w/w.

In particular the amount of waxes having a molecular weight of 1.25xMw to 2.0xMw is at least 15% w/w, e.g. at least 18% w/w, e.g. at least 20% w/w, e.g. at least 22% w/w, e.g. at least 24% w/w, e.g. at least 26% w/w, e.g. at least 28% w/w, e.g. at least 30% w/w, e.g. at least 35% w/w, e.g. at least 40% w/w.

In particular the amount of waxes having a molecular weight of 0.75xMw to 1.25xMw is at least 21% w/w, e.g. at least 22% w/w, e.g. at least 25% w/w, e.g. at least 28% w/w, e.g. at least 30% w/w, e.g. at least 35% w/w, e.g. at least 40% w/w, e.g. at least 45% w/w, e.g. at least 50% w/w.

In an alternative wording the coating comprises a wax composition obtainable by mixing two or more waxes with different molecular weights wherein at least two of the waxes have a molecular weight in the range of 0.25 x the average molecular weight of the wax composition to 2.0 x the average molecular weight of the wax composition.

The wax coating may preferably constitute less than 50% by weight of the total granule. More preferably the coating may constitute less than 30% by weight of the raw granule. In some cases the wax coating may constitute less than 10% or even 5% by weight of the total granule.

The wax coating of the present invention is substantially free of the enzyme.

Waxes

A "wax" in the context of the present invention is to be understood as a polymeric material having a melting point between 25 -150 °C, particularly 30 to100°C more particularly 35 to 85°C most particularly 40 to 75°C. The wax is preferably in a solid state at room temperature, 25°C. The lower limit is preferred to set a reasonable distance between the temperature at which the wax starts to melt to the temperature at which the granules or compositions comprising the granules are usually stored, 20 to 30°C.

The coating material of the invention is a wax composition. A "wax composition" in this context is to be understood as mixture comprising two or more waxes. Such compositions usually have a melting range rather than a melting point. The temperature at which the wax composition start to melt is called $T_{m,i}$, and the median melting point for the wax composition is called $T_{m,m}$, while the temperature at which all wax solids are melted is called $T_{m,e}$. The median melting point in this context is defined as the temperature at which 50% w/w of the solids in the wax are melted.

In a particular embodiment of the present invention the $T_{m,i}$ of the wax composition is more than 25°C. In a more particular embodiment of the present invention the $T_{m,i}$ of the wax composition is more than 30°C. In a most particular embodiment of the present invention the $T_{m,i}$ of the wax composition is more than 35°C.

In particular, suitable wax compositions for the present invention have a high median melting point $T_{m,m}$. The median melting point is particularly greater than or equal to 50°C, in particular $T_{m,m} \geq 51$ °C. e.g. $T_{m,m} \geq 52$°C, e.g. $T_{m,m} \geq 53$°C, e.g. $T_{m,m} \geq 54$°C, e.g. $T_{m,m} \geq 55$°C, e.g. $T_{m,m} \geq 56$°C, e.g. $T_{m,m} \geq 57$°C, e.g. $T_{m,m} \geq 58$°C, e.g. $T_{m,m} \geq 59$°C, e.g. $T_{m,m} \geq 60$°C, e.g. $T_{m,m} \geq 65$°C, e.g. $T_{m,m} \geq 70$°C, e.g. $T_{m,m} \geq 75$°C, e.g. $T_{m,m} \geq 80$°C, e.g. $T_{m,m} \geq 85$°C, e.g. $T_{m,m} \geq 90$°C, e.g. $T_{m,m} \geq 95$°C, e.g. $T_{m,m} \geq 100$°C. In particular the median melting point should be within or between 50 to 60 °C such as 51 to 60 °C

In particular, suitable wax compositions for the present invention have a broad melting range, which is a frequent characteristic of compositions having a broad molecular weight distribution. In particular the melting range for the wax composition is at least 10°C, e.g. at least 11°C. e.g. at least 12°C, e.g. at least 14°C, e.g. at least 16°C, e.g. at least 18°C, e.g. at least 20°C, e.g. at least 25°C, e.g. at least 30°C, e.g. at least 35°C, e.g. at least 40°C

The melting range is calculated as the difference in degrees celcius between the temperature at which all wax solids are melted ($T_{m,e}$) and the temperature at which the wax composition starts to melt ($T_{m,i}$).

For some granules, e.g. granules used in the detergent industry, a preferable feature of the wax is that the wax should be water soluble or water dispersible, particularly in neutral and alkaline solution, so that when the coated particles of the invention is introduced into an aqueous solution, i.e. by diluting it with water, the wax should disintegrate and/or dissolve providing a quick release and dissolution of the active incorporated in the particles to the aqueous solution. Examples of water soluble waxes are poly ethylene glycols (PEG's). Amongst water insoluble waxes, which are dispersible in an aqueous solution are triglycerides and oils. For some granules it is preferable that the coating contains some insoluble waxes e.g. feed granules.

Preferably the wax composition is a hydrophilic composition. In a particular embodiment at least 25 % w/w of the constituents comprised in the wax composition is soluble in water, preferably at least 50% w/w, preferably at least 75% w/w, preferably at least 85% w/w, preferably at least 95% w/w, preferably at least 99% w/w.

In another embodiment the wax composition is hydrophilic and dispersible in an aqueous solution.

In a particular embodiment the wax composition comprise less than 75% w/w hydrophobic constituents, preferably less than 50% w/w, preferably less than 25% w/w, preferably less than 15% w/w, preferably less than 5% w/w, preferably less than 1% w/w.

In a particular embodiment the wax composition comprise less than 75% w/w water insoluble constituents, preferably less than 50% w/w, preferably less than 25% w/w, preferably less than 15% w/w, preferably less than 5% w/w, preferably less than 1% w/w.

Suitable waxes are organic compounds or salts of organic compounds having one or more of the above mentioned properties.

The wax composition may preferably constitute at least 10 % by weight of the coating material, more preferably at least 20 % by weight of the coating material.

The wax composition of the invention may comprise any wax, which is chemically synthesized. It may also equally well comprise waxes isolated from a natural source or a derivative thereof. Accordingly, the wax composition of the invention may comprise waxes selected from the following non limiting list of waxes.

- Poly ethylene glycols, PEG. Different PEG waxes are commercially available having different molecular sizes, wherein PEG's with low molecular sizes also have low melting points. Examples of suitable PEG's are PEG 1500, PEG 2000, PEG 3000, PEG 4000, PEG 6000, PEG 8000, PEG 9000 etc. e.g. from BASF (Pluriol E series) or from Clariant or from Ineos. Derivatives of polyethylene glycols may also be used.

- polypropylens (e.g. polypropylen glycol Pluriol P series from BASF) or polyethylens or mixtures thereof. Derivatives of polypropylenes and polyethylenes may also be used.

- Nonionic surfactants which are solid at room temperature such as ethoxylated fatty alcohols having a high level of ethoxy groups such as the Lutensol AT series from BASF, a C16-C18 fatty alcohol having different amounts of ehtyleneoxide per molecule, e.g. Lutensol AT11, AT13, AT25, AT50, AT80, where the number indicate the average number of ethyleneoxide groups. Alternatively polymers of ethyleneoxide, propylene oxide or copolymers thereof are useful, such as in block polymers, e.g. Pluronic PE 6800 from BASF. Derivatives of ethoxylated fatty alcohols.

- Waxes isolated from a natural source, such as Carnauba wax (melting point between 80-88°C), Candelilla wax (melting point between 68-70°C) and bees wax. Other natural waxes or derivatives thereof are waxes derived from animals or plants, e.g. of marine origin. Hydrogenated plant oil or animal tallow. Examples of such waxes are hydrogenated ox tallow, hydrogenated palm oil, hydrogenated cotton seeds and/or hydrogenated soy bean oil, wherein the term "hydrogenated" as used herein is to be construed as saturation of unsaturated carbohydrate chains, e.g. in triglycerides, wherein carbon=carbon double bonds are converted to carbon-carbon single bonds. Hydrogenated palm oil is commercially available e.g. from Hobum Oele und Fette GmbH - Germany or Deutche Cargill GmbH - Germany.

- Fatty acid alcohols, such as the linear long chain fatty acid alcohol NAFOL 1822 (C18, 20, 22) from Condea Chemie GMBH - Germany, having a melting point between 55-60°C. Derivatives of fatty acid alcohols.

- Mono-glycerides and/or di-glycerides, such as glyceryl stearate, wherein stearate is a mixture of stearic and palmitic acid, are useful waxes. An example of this is Dimodan PM - from Danisco Ingredients, Denmark.

- Fatty acids, such as hydrogenated linear long chained fatty acids and derivatives of fatty acids.

- Paraffines, i.e. solid hydrocarbons.

- Micro-crystalline wax.

In further embodiments waxes which are useful in the invention can be found in C.M. McTaggart et. al., Int. J. Pharm. 19, 139 (1984) or Flanders et. al., Drug Dev. Ind. Pharm. 13, 1001 (1987) both incorporated herein by reference.

In a particular embodiment of the present invention the wax of the present invention is a mixture of two or more different waxes.

In a particular embodiment of the present invention the wax or waxes is selected from the group consisting of PEG, ethoxylated fatty alcohols, fatty acids, fatty acid alcohols and glycerides.

In another particular embodiment of the present invention the waxes are chosen from synthetic waxes. In a more particular embodiment the waxes of the present invention are PEG or nonionic surfactants. In a most particular embodiment of the present invention the wax is PEG.

*Molecular weight of the wax:*

Commercial wax compositions are often specifying an average molecular weight e.g. PEG 4000. Such numbers refers to the average molecular weight of the wax molecules in the product, and the molecular weights of the individual molecules present in the

but these given molecular weights are the average molecular weight of a narrow molecular weight distribution.

In a particular embodiment of the present invention the waxes or mixture of waxes have an average molecular weight of 500 to 20,000. In a more particular embodiment of the present invention the waxes or mixture of waxes have an average molecular weight of 750 to 15,000. In an even more particular embodiment of the present invention the waxes or mixtures of waxes have an average molecular weight of 1,000 to 10,000, e.g. 2,000 to 8,000, e.g. 3,000 to 6,000.

In a particular embodiment of the present invention the Mw is more than 1000. In a more particular embodiment of the present invention the Mw is more than 1200. In an even more particular embodiment of the present invention the Mw is more than 1400.

The wax composition comprising a wide molecular weight distribution may in particular be obtained by mixing two or more wax compositions each having a different average molecular weight and a, preferably narrow, approximately Poisson distributed molecular weight distribution. The resulting wax composition used for coating granules of the invention thus have a wider molecular weight distribution than the wax compositions used for preparing said wax composition, and the molecular weights of the resulting wax composition are non-Poisson distributed.

Alternatively, the wax composition comprising a wide molecular weight distribution may in particular be obtained by mixing two or more wax compositions each having a different median melting point and a, preferably narrow, approximately Poisson distributed melting point distribution. The resulting wax composition used for coating granules of the invention thus have a broader melting point distribution than the wax compositions used for preparing said wax composition, and the molecular weights of the resulting wax composition are non-Poisson distributed.

In particular the average molecular weights of the wax compositions used to prepare the wax composition of the invention are in the range 0.25 x Mw to 2.0 x Mw, where Mw is the average molecular weight of wax composition resulting from the mixture. In particularly, at least three wax compositions are used to prepare the wax composition of the invention, where at least two of the wax compositions are in the range 0.25 x Mw to 2.0 x Mw, where Mw is the average molecular weight of wax composition resulting from the mixture. Even more particularly, at least four wax compositions are used to prepare the wax composition of the invention, where at least two of the wax compositions are in the range 0.25 x Mw to 2.0 x Mw, where Mw is the average molecular weight of wax composition resulting from the mixture. Most particularly, at least five different wax compositions are used to prepare the wax composition of the invention, where at least two of the wax compositions are in the range 0.25 x Mw to 2.0 x Mw,

where Mw is the average molecular weight of wax composition resulting from the mixture.

In a particular embodiment of the present invention the granule comprises a core and a coating wherein the coating comprise a wax composition with a molecular weight distribution in the range:

at least 10%w/w + P(0.25xMw to 0.75xMw) in the range 0.25xMw to 0.75xMw, and
at least 20%w/w in the range 0.75xMw to 1.25xMw, and
at least 10%w/w + P(1.25xMw to 2.0xMw) in the range 1.25xMw to 2.0xMw or more preferred
at least 15% w/w + P(0.25xMw to 0.75xMw) in the range 0.25xMw to 0.75xMw, and
at least 20% w/w in the range 0.75xMw to 1.25xMw, and
at least 15% w/w + P(1.25xMw to 2.0xMw) in the range 1.25xMw to 2.0xMw or even more preferred
at least 20% w/w + P(0.25xMw to 0.75xMw) in the range 0.25xMw to 0.75xMw, and
at least 20% w/w in the range 0.75xMw to 1.25xMw, and
at least 20% w/w + P(1.25xMw to 2.0xMw) in the range 1.25xMwto 2.0xMw

Where P(interval) is defined as the weight percentage of molecules being in the interval, if the polymer is following a Poisson distribution, defined by the mean number of monomers Mw given by (Mw-18 g/mole)/(44 g/mole) rounded to nearest integer (i.e. the weight percentage of a Poisson distributed PEG polymer with weight average molecular weight Mw in the interval):

P(interval) = 100 x ( $F(n_k) \times M(n_k) + F(n_{k+1}) \times M(n_{k+1}) + .... + F(n_j) \times M(n_j)$ )/Mw, where F(n) is calculated as defined in the section "Definitions" and where $n_k$ is the number of repeating units in the molecule (as defined above for Mw) corresponding to the lowest Mw (rounded up to the nearest integer) in the interval and $n_j$ is the number of repeating units in the molecules corresponding to the highest Mw (rounded down to the nearest integer) in the interval.

These intervals, P(0.25xMw to 0.75xMw), ensure that the molecular weight distribution of the polymeric wax is significantly wider than a corresponding Poisson distributed polymer of a PEG-type.

Additional coating materials

The wax coating may further comprise additional coating materials such as fillers, binders, fibre materials, enzyme stabilizing agents, solubilising agents, crosslinking agents, suspension agents, viscosity regulating agents, light spheres, chlorine scavengers, plasticizers, pigments, salts, lubricants (such as surfactants or antistatic agents) and fragrances.

Fillers:

Suitable fillers are water soluble and/or insoluble inorganic salts such as finely ground alkali sulphate, alkali carbonate and/or alkali chloride, clays such as kaolin (e.g. SPESWHITE™, English China Clay), bentonites, talcs, zeolites, chalk, calcium carbonate and/or silicates.

Binders:

Suitable binders are binders with a high melting point or no melting point at all and of a non waxy nature e.g. polyvinyl pyrrolidon, dextrins, polyvinylalkohol, cellulose derivatives, for example hydroxypropyl cellulose, methyl cellulose or CMC. A suitable binder is a carbohydrate binder such as Glucidex 21 D™ available from Roquette Freres, France.

Fiber materials:

Pure or impure cellulose in fibrous form such as sawdust, pure fibrous cellulose, cotton, or other forms of pure or impure fibrous cellulose. Also, filter aids based on fibrous cellulose can be used. Several brands of cellulose in fibrous form are on the market, e.g. CEPO™ and ARBO-CELL™. Pertinent examples of fibrous cellulose filter aids are ARBO-CELL BFC 200™ and ARBOCELL BC 200™. Also synthetic fibres may be used as described in EP 304331 B1 and typical fibres may be made of polyethylene, polypropylene, polyester, especially nylon, polyvinylformat, poly(meth)acrylic compounds.

Enzyme stabilizing agents:

Enzyme stabilising or protective agents such as conventionally used in the field of granulation may be elements of the coating. Stabilising or protective agents may fall into several categories: alkaline or neutral materials, reducing agents, antioxidants and/or salts of first transition series metal ions. Each of these may be used in conjunction with other protective agents of the same or different categories. Examples of alkaline protective agents are alkali metal silicates, carbonates or bicarbonates, which provide a chemical scavenging effect by actively neutralising e.g. oxidants. Examples of reducing protective agents are salts of sulfite, thiosulfite, thiosulfate or $MnSO_4$ while examples of antioxidants are methionine, butylated hydroxytoluene (BHT) or butylated hydroxyanisol (BHA). In particular stabilising agents may be salts of thiosulfates, e.g. sodium thiosulfate or methionine. Also enzyme stabilizers may be borates, borax, formates, di- and tricarboxylic acids and reversible enzyme inhibitors such as organic compounds with sulfhydryl groups or alkylated or arylated boric acids. Examples of boron based stabilizer may be found in WO 96/21716, whereas a preferred boron based stabilizer is 4-Formyl-Phenyl-Boronic Acid or derivatives thereof described in WO 96/41859 both disclosures incorporated herein by reference. Still other examples of useful enzyme stabilizers are gelatine, casein, Polyvinylpyrrolidone (PVP) and powder of skimmed milk. The amounts of protective agent in the coating may be 5-40% w/w of the coating, particularly 5-30%, e.g. 10-20%.

Solubilising agents:

The solubility of the coating is especially critical in cases where the coated particle is a component of a detergent formulation. As is known by the person skilled in the art, many agents, through a variety of methods, serve to increase the solubility of formulations, and typical agents known to the art can be found in National Pharmacopeia's.

Light spheres:

Light spheres are small particles with low true density. Typically, they are hollow spherical particles with air or gas inside. Such materials are usually prepared by expanding a solid material. These light spheres may be inorganic of nature such as SCOTCHLITE□ Glass Bubbles from 3M□ (hollow glass spheres), Q-CEL□ (hollow microspheres of borosilicate glass) and/or Extendospheres□ (ceramic hollow spheres) available from The PQ Corporation. The light spheres may also be of organic nature such as the PM-series (plastic hollow spheres) available from The PQ Corporation. Expancel□ (hollow plastic spheres) from AKZO Nobel, Luxsil□ and Sphericel□ from Potters Industries and/or Styrocell□ from SHELL, which is spheres of polystyrene. The polystyrene of Styrocell□ contains pentane which upon heating boils and expands or pops the material (the reaction is comparable to the expansion of corn seeds into popcorn) leaving a light polystyrene material of a low true density. Also polysaccharides are preferred, such as starch or derivatives thereof. Biodac□ is an example of non-hollow lightweight material made from cellulose (waste from papermaking), available from GranTek Inc. These materials may be included in the granules of the invention either alone or as a mixture of different light materials.

Crosslinking agents:

Cross-linking agents such as enzyme-compatible surfactants, e.g. ethoxylated alcohols, especially ones with 10 to 80 ethoxy groups. These may both be found in the coating and in the core particle.

Suspension agents:

Suspension agents, mediators (for boosting bleach action upon dissolution of the particle in e.g. a washing application) and/or solvents may be incorporated in the coating.

Viscosity regulating agents:

Viscosity regulating agents may be present in the coating.

Plasticizers:

Plasticizers useful in coating layers in the context of the present invention include, for example: polyols such as sugars, sugar alcohols, glycerine, glycerol trimethylol propane, neopentyl glycol, triethanolamine, mono-, di- and triethylene glycol or polyethylene glycols (PEGs) having a molecular weight less than 1000; urea, phthalate esters such as dibutyl or dimethyl phthalate; thiocyanates, non-ionic surfactants such as ethoxylated alcohols and ethoxylated phosphates and water.

Pigments:

Suitable pigments include, but are not limited to, finely divided whiteners, such as titanium dioxide or kaolin, coloured pigments, water soluble colorants, as well as combinations of one or more pigments and water soluble colorants. In case where the granules are comprising enzymes and the granules are additives for detergents, a whitening agent e.g. $TiO_2$ can be incorporated in the granules. By adding the $TiO_2$ at different times during the granulation process, if the granulation is performed discontinuously, at different positions in the in the granulator, or if the granulation is performed continuously, the $TiO_2$ may be distributed inside the wax coating or on the surface of the wax coating or both if desired.

Salts:

The salt may be an inorganic salt, e.g. salts of sulfate, sulfite, phosphate, phosphonate, nitrate, chloride or carbonate or salts of simple organic acids (less than 10 carbon atoms e.g. 6 or less carbon atoms) such as citrate, malonate or acetate. Examples of cations in these salt are alkali or earth alkali metal ions, although the ammonium ion or metal ions of the first transition series, such as sodium, potassium, magnesium, calcium, zinc or aluminium. Examples of anions include chloride, bromide, iodide, sulfate, sulfite, bisulfite, thiosulfate, phosphate, monobasic phosphate, dibasic phosphate, hypophosphite, dihydrogen pyrophosphate, tetraborate, borate, carbonate, bicarbonate, metasilicate, citrate, malate, maleate, malonate, succinate, lactate, formate, acetate, butyrate, propionate, benzoate, tartrate, ascorbate or gluconate. In particular alkali- or earth alkali metal salts of sulfate, sulfite, phosphate, phosphonate, nitrate, chloride or carbonate or salts of simple organic acids such as citrate, malonate or acetate may be used. Specific examples include $NaH_2PO_4$, $Na_2HPO_4$, $Na_3PO_4$, $(NH_4)H_2PO_4$, $K_2HPO_4$, $KH_2PO_4$, $Na_2SO_4$, $K_2SO_4$, $KHSO_4$ $ZnSO_4$, $MgSO_4$ $CuSO_4$, $Mg(NO_3)_2$, $(NH_4)_2SO_4$, sodium borate, magnesium acetate and sodium citrate.
The salt may also be a hydrated salt, i.e. a crystalline salt hydrate with bound water(s) of crystallization, such as described in WO 99/32595. Examples of hydrated salts include magnesium sulfate heptahydrate ($MgSO_4(7H_2O)$) zinc sulfate heptahydrate ($ZnSO_4(7H_2O)$), copper sulfate pentahydrate ($CuSO_4(5H_2O)$), sodium phosphate dibasic heptahydrate ($Na_2HPO_4(7H_2O)$), magnesium nitrate hexahydrate ($Mg(NO_3)_2(6H_2O)$), sodium borate decahydrate, sodium citrate dihydrate and magnesium acetate tetrahydrate.

Lubricant:

As used in the present context, the term "lubricant" refers to any agent, which reduces surface friction, lubricates the surface of the granule, decreases tendency to build-up of static electricity, and/or reduces friability of the granules. Lubricants can also play a related role in improving the coating process, by reducing the tackiness of binders in the coating. Thus, lubricants can serve as anti-agglomeration agents and wetting agents. Examples of suitable lubricants are lower polyethylene glycols (PEGs), ethoxylated fatty alcohols and mineral oils. The lubricant is particularly a mineral oil or a nonionic surfactant, and more particularly the lubricant is not miscible with the other coating materials.

**The core**

The particles to be coated with the wax coating of the present invention may be of any type known to the art.

Generally all kinds of particles comprising an active compound having a size of 50 to 2000 μm more particularly 250 to 1500 μm even more particularly 400 to 750 μm may be coated in accordance with the present invention.

The core particle contains the enzyme. Besides of the enzyme the core particle may be constructed in any way or of any material, which provides the desired functional properties of the core material, e.g. the core may consist of materials, which allows readily release of the enzymes upon introduction to an aqueous medium. In one embodiment the core particle is constructed of a particulate carrier where the enzyme is absorbed into the carrier, in another embodiment the core particle is constructed of a particulate carrier where an enzyme containing layer is applied on the carrier surface, optionally comprising a protecting reducing agent. There may even be additional coating within the core material providing desired functional properties of the core material. Another core particle may be the so called T-granulate wherein enzymes and granulation material is mixed to form granules incorporating the enzyme distributed throughout the core such as described in US 4,106,991 e.g. Example 1. Any conventional methods and non-active materials may be used to prepare the core particle. Examples of known conventional cores particles and materials is, inter alia, described in, US 4,106,991 (in particular), EP 170360, EP 304332, EP 304331, EP 458849, EP 458845, WO 97/39116, WO 92/12645, WO 89/08695, WO 89/08694, WO 8 7/07292, WO 91/06638, WO 9 2/13030, WO 93/07260, WO 9 3/07263, WO 9 6/38527, WO 96/16151, WO 97/23606, WO 01/23513, US 5,324,649, US 4,689,297, EP 206417, EP 193829, DE 4344215, DE 4322229 A, DD 263790, JP 61162185 A, JP 58179492.

As a particularly embodiment of the invention the core particle may be prepared by applying a layer of enzyme onto a non-pareil or placebo carrier (active-free carrier) according to the methodology described in U S 5 ,324,649. Optionally additional active compound may be absorbed into the surface of the carrier.

In a particular embodiment of the invention the core particle may also comprise a protective agent or enzyme stabilizing agent as described for the coating, vide supra, particularly mixed with the active compound in suitable amounts such as 0.1-5% w/w. of the coated particle, particularly 0.2-3% w/w, e.g. 0.3-2% w/w. The protective agent may be an antioxidant, a reducing agent or a mixture.

In a particular embodiment of the present invention the core particle may comprise an active compound e.g. an enzyme stabilized by the addition of a synthetic polymer and an antioxidant or reducing agent as described in WO-A-2004/003188. The visco-elastic liquid making up the matrix wherein the active and optionally other useful components may in principle be any material or mixtures of materials which meets the requirements for viscosity and elasticity set for the visco-elastic core particles, as described in WO02/28991

In particular materials may be organic visco-elastic materials such as liquid materials comprising, consisting of or containing organic polymers and/or monomers. Materials such as carbohydrate polymers (e.g. pectins), proteins (e.g.) gelatin, sugars, glucose syrups, modified vegetable oils or mixtures thereof can be brought or formulated into a liquid state having visco-elastic properties as described above,

Particularly a majority of the components constituting the visco-elastic liquid matrix are water soluble.

Active compound

The active compound of the invention is an enzyme. As said the active may be present dispersed as discrete solid particles in the core particle.

Enzymes:

The enzyme in the context of the present invention may be any enzyme or combination of different enzymes, Accordingly, when reference is made to "an enzyme" this will in general be understood to include combinations of one or more enzymes.

It is to be understood that enzyme variants (produced, for example, by recombinant techniques) are included within the meaning of the term "enzymes". Examples of such enzyme variants are disclosed, e.g., in EP 251,446 (Genencor), WO 91/00345 (Novo Nordisk), EP 525,610 (Solvay) and WO 94/02618 (Gist-Brocades NV).

The enzyme classification employed in the present specification with claims is in accordance with Recommendations (1992) of the Nomenclature Committee of the International Union of Biochemistry and Molecular Biology, Academic Press, Inc., 1992.

Accordingly the types of enzymes which may appropriately be incorporated in granules of the invention include oxidoreductases (EC 1.-.-.-), transferases (EC 2.-.-.-), hydrolases (EC 3.-.-.-), lyases (EC 4.-.-.-), isomerases (EC 5.-.-.-) and ligases (EC 6.-.-.-).

Preferred oxidoreductases in the context of the invention are peroxidases (EC 1.11.1), laccases (EC 1.10.3.2) and glucose oxidases (EC 1.1.3.4)]. An Example of a commercially available oxidoreductase (EC 1.-.-.-) is GLUZYME™ (enzyme available from Novozymes A/S). Further oxidoreductases are available from other suppliers. Preferred transferases are transferases in any of the following sub-classes:

a) Transferases transferring one-carbon groups (EC 2.1);
b) transferases transferring aldehyde or ketone residues (EC 2.2); acyltransferases (EC 2.3);
c) glycosyltransferases (EC 2.4);
d) transferases transferring alkyl or aryl groups, other that methyl groups (EC 2.5); and
e) transferases transferring nitrogeneous groups (EC 2.6).

A most preferred type of transferase in the context of the invention is a transglutaminase (protein-glutamine γ-glutamyltransferase; EC 2.3.2.13)..

Further examples of suitable transglutaminases are described in WO 96/06931 (Novo Nordisk A/S).

Preferred hydrolases in the context of the invention are: Carboxylic ester hydrolases (EC 3.1.1.-) such as lipases (EC 3.1.1.3); phytases (EC 3.1.3.-), e.g. 3-phytases (EC 3.1.3.8) and 6-phytases (EC 3.1.3.26); glycosidases (EC 3.2, which fall within a group denoted herein as "carbohydrases"), such as α-amylases (EC 3.2.1.1); peptidases (EC 3.4, also known as proteases); and other carbonyl hydrolases].

In the present context, the term "carbohydrase" is used to denote not only enzymes capable of breaking down carbohydrate chains (e.g. starches or cellulose) of especially five- and six-membered ring structures (i.e. glycosidases, EC 3.2), but also enzymes capable of isomerizing carbohydrates, e.g. six-membered ring structures such as D-glucose to five-membered ring structures such as D-fructose.

Carbohydrases of relevance include the following (EC numbers in parentheses):

α-amylases (EC 3.2.1.1), β-amylases (EC 3.2.1.2), g lucan 1,4-α-glucosidases (EC 3.2.1.3), endo-1,4-beta-glucanase (cellulases, EC 3.2.1.4), endo-1,3(4)-β-glucanases (EC 3.2.1.6), endo-1,4-β-xylanases (EC 3.2.1.8), dextranases (EC 3.2.1.11), chitinases (EC 3.2.1.14), polygalacturonases (EC 3.2.1.15), lysozymes (EC 3.2.1.17), β-glucosidases (EC 3.2.1.21), α-galactosidases (EC 3.2.1.22), β-galactosidases (EC 3.2.1.23), amylo-1,6-glucosidases (EC 3.2.1.33), xylan 1,4-β-xylosidases (EC 3.2.1.37), glucan endo-1,3-β-D-glucosidases (EC 3.2.1.39), α-dextrin endo-1,6-α-glucosidases (EC3.2.1.41), sucrose α-glucosidases (EC 3.2.1.48), glucan endo-1,3-α-glucosidases (EC 3.2.1.59), glucan 1,4-β-glucosidases (EC 3.2.1.74), glucan endo-1,6-β-glucosidases (EC 3.2.1.75), arabinan endo-1,5-α-L-arabinosidases (EC 3.2.1.99), lactases (EC 3.2.1.108), chitosanases (EC 3.2.1.132) and xylose isomerases (EC 5.3.1.5).

Examples of commercially available proteases (peptidases) include KAN-NASE™, EVERLASE™, ESPERASE™, ALCALASE™, NEUTRASE™, DURAZYM™, SAVI-NASE™, PYRASE™, Pancreatic Trypsin NOVO (PTN), BIO-FEED™ PRO and CLEAR-LENS™ PRO (all available from Novozymes A/S).

Other commercially available proteases include MAXATASE™, MAXACAL™, MAXAPEM™, OPTICLEAN™ and PURAFECT™ (available from Genencor International Inc. or DSM).

Examples of commercially available lipases include LIPOPRIME™ LIPO-LASE™, LIPOLASE™ Ultra, LIPOZYME™, PALATASE™, NOVOZYM™ 435 and LECITASE™ (all available from Novozymes A/S).

Other commercially available lipases include LUMAFAST™ (*Pseudomonas mendocina* lipase from Genencor International Inc.); LIPOMAX™ (*Ps. pseudoalcaligenes* lipase from DSM/Genencor Int. Inc.; and *Bacillus* sp. lipase from Genencor enzymes. Further lipases are available from other suppliers.

Examples of commercially available carbohydrases include ALPHA-GAL™, BIO-FEED™ Alpha, BIO-FEED™ Beta, BIO-FEED™ Plus, BIO-FEED™ Plus, NOVOZYME™ 188, CELLUCLAST™, CELLUSOFT™, CEREMYL™, CITROZYM™, DENIMAX™, DEZYME™, DEXTROZYME™, FINIZYM™, FUNGAMYL™, GAMANASE™, GLUCANEX™, LACTOZYM™, MALTOGENASE™, PENTOPAN™, PECTINEX™, PROMOZYME™, PULPZYME™, NOVA-MYL™, TERMAMYL™, AMG™ (Amyloglucosidase Novo), MALTOGENASE™, SWEETZYME™ and AQUAZYM™ (all available from Novozymes A/S). Further carbohydrases are available from other suppliers.

Additional coatings

The granule of the present invention may comprise one or more additional coatings besides the wax coating.

Additional coatings may be present either in between the core and the wax coating or as a layer outside the wax coating.

Conventional coatings and methods as known to the art may suitably be used, such as the coatings described in Danish PA 2002 00473, Danish PA 2001 01930, WO 89/08694, WO 89/08695, 270 608 B1 and/or WO 00/01793. Other examples of conventional coating materials may be found in US 4,106,991, EP 170360, EP 304332, EP 304331, EP 458849, EP 458845, WO 97/39116, WO 92/12645A, WO 89/08695, WO 89/08694, WO 87/07292, WO 91/06638, WO 92/13030, WO 9 3/07260, WO 9 3/07263, WO 96/38527, WO 96/16151, WO 97/23606, WO 01/25412, WO 02/20746, WO 02/28369, US 5879920, US 5,324,649, US 4,689,297, US 6,348,442, EP 206417, EP 193829, DE 4344215, DE 4322229 A, DE 263790, JP 61162185 A and/or JP 58179492.

In a particular embodiment of the present invention the additional coating layer is a salt coating as described in DK 02/00885, hereby incorporated by reference

Conventional coating materials may be used including the materials mentioned under the section "The coating".

In a particular embodiment of the present invention the coating layers around the core unit have a thickness such as the ratio between the diameter of the granule and the diameter of the core being at least 1.1 as described in WO 01/25412, hereby incorporated by reference.

**Process for preparing granules**

The wax coasting:

In one embodiment of the present invention the process of preparing the granules of the present invention comprise contacting a particle comprising a n active compound with a coating, wherein the coating comprises a wax composition obtainable by: mixing two or more waxes with different molecular weights wherein at least two of the waxes have a molecular weight in the range of 0.25 x the average molecular weight of the wax composition to 2.0 x the average molecular weight of the wax composition.

In another embodiment of the present invention the process of preparing the granules of the present invention comprise contacting a particle comprising an active compound with a coating of the invention in particular, wherein the coating comprises a wax composition with a molecular weight distribution in the range of:

(a) at least 10% w/w in the range 0.25xMw to 0.75xMw, and
(b) at least 20% w/w in the range 0.75xMw to 1.25xMw, and
(c) at least 10% w/w in the range 1.25xMw to 2.0xMw,

where Mw is the weight average molecular weight of the wax composition.

In a particular embodiment of the present invention the contacting of the particle with the wax coating is taking place in a coating chamber.

In a particular embodiment of the present invention the contacting of the particle with the wax coating is taking place in a fluid bed apparatus or in a mixer apparatus.

The waxes used in the wax coating are always used in liquid form in the coating process; the waxes are therefore either dissolved or dispersed in water or melted. If the waxes are dispersed in water, the water is removed during a drying step. If no water is used in the coating, usually no drying is needed. In this case the coating material only comprises melted wax, only cooling is needed to solidify the granules. In most cases, drying or cooling is usually carried out in a fluid bed. A flow conditioner or anticaking agent may be added to the granulate either before or after the cooling step, e.g. fumed silica, for instance the commercial products AEROSIL or CABOSIL.

The coating may be applied to the core particles using any conventional coating method including those preparation methods mentioned vide infra under the section "Preparing core particles. In a particular embodiment of the present invention the coating is applied in a mixer, in a fluid bed or in a pan or drum coater. In a particular embodiment of the fluid bed coating process, the fluidised core particles are sprayed with a solution containing the coating material (s) and the coating is deposited on the surface of the core particles by evaporating the solution solvent, see e.g. US 6,136,772.

If the coating is applied to the core particles in a fluid bed the temperature of the coating will typically be between 0 to 100°C, particularly between 10 to 90°C, more particularly between 10 to 80°C or most particularly between 10 to 70°C. The inlet air-temperature in the fluid bed will typically be between 30 to 200°C, particularly between 40 to 150°C, more particularly between 40 to 120°C.

As the coating comprises a wax and if it is applied in a mixer the mixer temperature should be higher than the melting temperature of the wax. This will typically lie between 30 to 100°C.

The coating process can take place in any of the known types of mixing granulators, drum granulators, pan granulators or modifications of these. If a mixing granulator is used, for example a mixing drum from the German Company Gebr. Lodige Maschinen G.m.b.H, it is preferred that small rotating knives are mounted in the granulator in order to compact the granules.

The granulation may be performed as described in US patent 4,106,991 and WO 92/12645 (hereby incorporated as references).

The coated granulates may be further coated with additional layers as mentioned vide supra, e.g. a colored layer such as $TiO_2$ or lubricated, or the particle comprising the active compound may be coated with one or more additional layers before applying the wax coating.

Preparing core particles

Methods for preparing core particles include those disclosed in the above mentioned references, and may include but are not limited to preparation methods like spray drying, layering, absorbtion, crystallization, precipitation extrusion, pelletizing, prilling and mixer granulation.

Methods for preparing a visco-elastic liquid core particle include those described in WO 02/28991.

**Compositions comprising the coated particle and their application**

The invention also relates to compositions comprising the granules of the invention. The composition may be any composition, but particularly the compositions are well suited for use in the pharmaceutical industry, the detergent industry, the feed and fodder industry and in the baking industry. Accordingly the compositions may be detergent compositions, a feed composition, a fodder composition, a baking composition or an additive to be incorporated in such compositions. The granules may further be used in fertilizer compositions or as a fertilizer and(or in combination with fertilizing agents.

Feed/Fodder

In a particular embodiment of the present invention the granules of the present invention are useful in animal feed compositions.

In a particular embodiment of the present invention the granules of the present invention are useful in fodder compositions.

Baking

In a special embodiment of the invention we have found that our development of coated granules comprising an active is useful in the baking industry. In a particular embodiment of the present invention the granules are used as dough improver.

Within the flour mill and the baking industry the use of active compounds, such as enzymes, is well established. Accordingly the invention provides baking compositions comprising the coated granules of the invention, in particular dough improver compositions or flour compositions comprising the dough improver.

When using enzymes in the baking industry certain enzyme activities are preferred. Flour has varying content of amylases leading to differences in the baking quality. Addition of amylases can be necessary in order to standardize the flour. Amylases and pentosanases generally provide sugar for the yeast fermentation, improve the bread volume, retard retro gradation, and decrease the staling rate and stickiness that results from pentosan gums. Examples of carbohydrases are given below.

Certain maltogenic amylases e.g. Novamyl™ can be used for prolonging the shelf life of bread for two or more days without causing gumminess in the product. Selectively modifies the gelatinized starch by cleaving from the non-reducing end of the starch molecules, low molecular weight sugars and dextrins. The starch is modified in such a way that retro gradation is less likely to occur. The produced low-molecular-weight sugars improve the baked goods water retention capacity without creating the intermediate-length dextrins that result in gumminess in the finished product. The enzyme is inactivated during bread baking, so it can be considered a processing aid, which does not have to be declared on the label.

The bread volume can be improved by fungal $\alpha$-amylases, which further provide good and uniform structure of the bread crumb.

Said $\alpha$-amylases are endoenzymes that produce maltose, dextrins and glucose. Cereal and some bacterial $\alpha$-amylases are inactivated at temperatures above the gelatinization temperature of starch, therefore when added to wheat dough it results in a low bread volume and a sticky bread interior. Fungamyl has the advantage of being thermolabile and is inactivated just below the gelatinization temperature.

Enzyme preparations containing a number of pentosanase and hemi-cellulase activities can improve the handling and stability of the dough, and improves the freshness, the crumb structure and the volume of the bread.

By hydrolysing the pentosans fraction in flour, it will lose a great deal of its water-binding capacity, and the water will then be available for starch and gluten. The gluten becomes more pliable and extensible, and the starch gelatinize more easily. Pentosanases can be used in combination with or as an alternative to emulsifiers.

Detergents

The coated particles of the invention may be added to and thus become a component of a detergent composition.

The detergent composition of the invention may for example be formulated as laundry detergent composition for hand or machine washings including a cleaning additive composition suitable for pre-treatment of stained fabrics or a fabric softener composition, or a detergent composition for use in general household hard surface cleaning operations, or a composition for hand or machine dishwashing operations.

In a specific aspect, the invention provides a detergent additive comprising the coated particles of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 a nd s ubtilisin 1 68 (described in WO 8 9/06279). Examples of trypsin-like proteases a re trypsin (e.g. of porcine or bovine origin) and the Fusarium protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include EVERLASE™, OVOZYME™, SAVOZYME™, ALCALASE™, SAVINASE™, PRIMASE™, DURALASE™, ES-PERASE™, AND KANNASE™ (Novozymes AIS), MAXATASE™, MAXACAL™, MAXAPEM™, PROPERASE™, PURAFECT™, PURAFECT OXP™, FN2™, and FN3™, FN4™ (Genencor International Inc.).

Lipases: Suitable lipases include those of bacterial or fungal origin. Chemically modified or protein engineered mutants are included. Examples of useful lipases include lipases from Humicola (synonym Thermomyces), e.g. from H. lanuginosa (T. lanuginosus) as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD. 705 (WO 95/06720 and WO 96/27002), P. wisconsinensis (WO 96/12012), a Bacillus lipase, e.g. from B. subtilis (Dartois et al. (1993), Biochemica et Biophysica Acta, 1131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include LIPOLASE™ and LIPOLASE ULTRA (Novozymes A/S).

Amylases: Suitable amylases ($\alpha$ and/or $\beta$) include those of bacterial or fungal origin. Chemically modified or protein engineered mutants are included. Amylases include, for example, $\alpha$-amylases obtained from Bacillus, e.g. a special strain of B. licheniformis, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156. 181, 188, 190, 197, 202, 208, 209, 243, 264, 304, 305, 391, 408, and 444.

Commercially available amylases are DURAMYL™, TERMAMYL™, FUNGA-MYL™ and BAN™ (Novozymes A/S), RAPIDASE™, PURASTAR™ and PURASTAR OXAM™ (from Genencor International Inc.).

Cellulases: Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include CELLUZYME™, and CAREZYME™ (Novozymes A/S), CLAZINASE™, and PURADAX HA™ (Genencor International Inc.), and KAC-500(B)™ (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from Coprinus, e.g. from C. cinereus, and variants thereof as those described in WO 93/24618, WO 95/10602, and WO 98/15257.

Commercially available peroxidases include GUARDZYME™ (Novozymes A/S). Suitable mannanases include MANNAWAY™ (Novozymes A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, may be formulated so as to contain one or more of the particles of the invention comprising different enzymes.

The detergent composition of the invention may be in any convenient dry form, e.g., a bar, a tablet, a powder, a granule or a paste. It may also be a liquid detergent, in particular non-aqueous liquid detergent.

The detergent composition comprises one or more surfactants, which may be non-ionic including semi-polar and/or anionic and/or cationic and/or zwitterionic. The surfactants are typically present at a level of from 0.1 % to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system, which may comprise a $H_2O_2$ source such as perborate or percarbonate, which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, may be added in an amount corresponding to 0.01-100 mg of enzyme protein per litre of wash liquor, preferably 0.05-5 mg of enzyme protein per litre of wash liquor, in particular 0.1-1 mg of enzyme protein per litre of wash liquor.

The enzyme of the invention may additionally be incorporated in the detergent formulations disclosed in WO 97/07202, which is hereby incorporated as reference.

## EXAMPLES

### Example 1

In the following:

Interval 1 is defined as from $0.25 \times Mw_{,mix}$ to $0.75 \times Mw_{,mix}$

Interval 2 is defined as from $0.75 \times Mw_{mix}$ to $1.25 \times Mw_{,mix}$

Interval 3 is defined as from 1.25xMw,mix to 2.00xMw,mix

Examples of mixtures P1 to P7 (calculated from Poisson distributions of polymers)

| PEG mixtures | P1 | P2 | P3 | P4 | P5 | P6 | P7 |
|---|---|---|---|---|---|---|---|
| PEG-400 | 10% | | | | | | |
| PEG-1000 | | | | | 2% | 10% | 9% |
| PEG-2000 | | | | 1% | 15% | 15% | 18% |
| PEG-3000 | | | 9% | 24% | 25% | 25% | 22% |
| PEG-4000 | 90% | 100% | 90% | 62% | 30% | 40% | 29% |
| PEG-6000 | | | 1% | 13% | 28% | 10% | 18% |
| PEG-8000 | | | | | | | 4% |
| Mw,mix | 3660 | 4022 | 3949 | 4012 | 3956 | 3356 | 3673 |
| $n_{w,mix}$ | 83 | 91 | 89 | 91 | 89 | 76 | 83 |
| Int.1 | 0% | 1% | 4% | 13% | 26% | 24% | 29% |
| Int. 2 | 81% | 98% | 93% | 74% | 45% | 52% | 44% |
| Int. 3 | 9% | 1% | 3% | 13% | 28% | 21% | 21% |
| Visual evaluation of crystalline structure | Large crystals | Large crystals | Large crystals | Medium Crystals/ amorph | Medium Crystals/ Amorph | Small Amorph | Small Amorph |
| Surface evaluation | Wet/sticky | Dry | Dry | Dry | Dry | Dry | Dry |

The crystalline structure (given by visual inspection of the size of the crystal patterns on the surface of solidified (cooled to room temperature) wax mixture) is less pronounced (more amorphous) with increasing wideness of molecular weights. Adding a liquid (at room temperature) wax to a solid (example P1) gives a wet and sticky surface (also making it difficult to evaluate crystal patterns).

**Example 2**

Two batches (one in a 130 liter Lödige mixer and one produced in a 600 liter Lödige mixer) of uncoated Savinase® (protease) granulate with an approximate activity of 15 KNPU/g were produced as described in US 4,106,991 example 1 with the following exceptions:

• Finely grinded sodium sulfate was used in stead of sodium chloride as filler material
• The enzyme concentrate (added as a liquid) contained also a carbohydrate binder (Avedex W80 dextrin)

The granulates were sieved in the fraction 250 to 850 microns and coated (in a 5 liter Lödige mixer) with 23.75% coating (relative to the uncoated material) as described in US 4,109,991 example 22 with the following coating compositions:

Reference coating: 41% PEG-4000, 41% Titan dioxide and 18% Calcium carbonate
PEG-mix coating: 41% Titan dioxide, 18% Calcium carbonate and 41% PEG-mixture, P6 from example 1, comprising: 10% PEG-1000, 15% PEG-2000, 25% PEG-3000, 40% PEG-4000, 10% PEG-6000.

All granulates were finally powdered with 0.75% Kaolin
The dust from the four granulates were tested using the conventional Heubach attrition method which is known to the art, measuring both the total dust created (as mg dust per 20 g granulate) and the enzyme dust (as nanogram protein per g granulate).

| Granulate | Total dust as mg per 20 gram granulate | Active dust as ng Savinase protein per gram granulate |
|---|---|---|
| Reference coating on 130 liter Lödige granulate | 74.9 | 1374 |
| Coating of the invention on 130 liter Lödige granulate | 0.0 | 13 |
| Reference coating on 600 liter Lödige granulate | 21.5 | 3090 |
| Coating of the invention on 600 liter Lödige granulate | 0.1 | 156 |

It is clearly seen that the coating of the invention reduces the dust significantly.

**Claims**

1. A granule comprising a core and a coating wherein the core comprises an enzyme and the coating comprises a wax composition with a molecular weight distribution of:

    (a) at least 10% w/w in the range $0.25 \times M_w$ to $0.75 \times M_w$, and
    (b) at least 20% w/w in the range $0.75 \times M_w$ to $1.25 M_w$, and
    (c) at least 10% w/w in the range $1.25 M_w$ to $2.0 \times M_w$, where $M_w$ is the weight average molecular weight of the wax composition.

2. The granule according to claim 1, wherein the temperature at which the wax composition starts to melt, $T_{m,i}$, is at least 25°C.

3. The granule of any preceding claim, wherein $T_{m,i}$ of the wax composition is at least 30°C.

4. The granule of any preceding claim, wherein $T_{m,i}$ of the wax composition is at least 35°C.

5. The granule of any preceding claim, wherein the median melting point is between 50 to 60 °C

6. The granule of any preceding claim, wherein the melting range is at least 10°C.

7. The granule of any preceding claim, wherein $M_w$ is more than 1000.

8. The granule of any preceding claim, wherein $M_w$ is more than 1200.

9. The granule of any preceding claim, wherein $M_w$ is more than 1400.

10. The granule of any preceding claim, wherein the waxes are selected from the group consisting of PEG, ethoxylated fatty alcohols, fatty acids, fatty acid alcohols and glycerides.

11. The granule of any preceding claim, wherein the granules have a caking strength of less than 1000.

12. A process for preparing a granule of claims 1-11, comprising contacting a particle comprising an active compound with a coating, wherein the coating comprises a wax composition with a molecular weight distribution in the range of:

    (a) at least 10% w/w in the range 0.25xMw to 0.75xMw, and
    (b) at least 20% w/w in the range 0.75xMw to 1.25xMw, and
    (c) at least 10% w/w in the range 1.25xMw to 2.0xMw,

where Mw is the weight average molecular weight of the wax composition.

**13.** The process of claim 12, wherein said contacting of the particle with a coating is taking place in a coating chamber.

**14.** The process of claim 12, wherein said contacting of the particle with a coating is taking place in a fluid bed apparatus or in a mixer apparatus.

**15.** A feed or fodder composition for animals comprising the granule of claims 1 to 11.

**16.** A method of preparing the feed or fodder composition of claim 15, comprising mixing a granule of claims 1-11 with feed or fodder composition.

**17.** A dough composition comprising the granule of claims 1 to 11.

**18.** A method of improving a dough composition comprising mixing a dough composition with a granule of claims 1-11.

**19.** A detergent composition comprising a granule of claims 1-11.

**20.** A method of preparing a detergent composition comprising mixing a granule of claims 1-11 with a surfactant.

**21.** A fertilizer composition comprising the granule of claims 1 to 11.

**22.** A method of preparing a fertilizer composition comprising mixing a granule of claims 1-11 with a fertilizing agent

**23.** A pharmaceutical composition comprising the granule of claims 1 to 11.


**Patentansprüche**

**1.** Granalie, umfassend einen Kern und eine Umhüllung, wobei der Kern ein Enzym umfasst und die Umhüllung eine Wachszusammensetzung mit einer Molekulargewichtsverteilung umfasst von:

(a) mindestens 10% (w/w) in dem Bereich $0,25 \times M_w$ bis $0,75 \times M_w$, und
(b) mindestens 20% (w/w) in dem Bereich $0,75 \times M_w$ bis $1,25 \times M_w$, und
(c) mindestens 10% (w/w) in dem Bereich $1,25 \times M_w$ bis $2,0 \times M_w$, wobei $M_w$ das Durchschnittsgewicht des Molekulargewichts der Wachszusammensetzung ist.

**2.** Granalie nach Anspruch 1, wobei die Temperatur, bei der die Wachszusammensetzung anfängt zu schmelzen, $T_{m,i}$, mindestens 25°C beträgt.

**3.** Granalie nach einem beliebigen vorangehenden Anspruch, wobei die $T_{m,i}$ der Wachszusammensetzung mindestens 30°C beträgt.

**4.** Granalie nach einem beliebigen vorangehenden Anspruch, wobei die $T_{m,i}$ der Wachszusammensetzung mindestens 35°C beträgt.

**5.** Granalie nach einem beliebigen vorangehenden Anspruch, wobei der mittlere Schmelzpunkt zwischen 50 bis 60°C beträgt.

**6.** Granalie nach einem beliebigen vorangehenden Anspruch, wobei der Schmelzbereich mindestens 10°C beträgt.

**7.** Granalie nach einem beliebigen vorangehenden Anspruch, wobei $M_w$ mehr als 1000 beträgt.

**8.** Granalie nach einem beliebigen vorangehenden Anspruch, wobei $M_w$ mehr als 1200 beträgt.

**9.** Granalie nach einem beliebigen vorangehenden Anspruch, wobei $M_w$ mehr als 1400 beträgt.

**10.** Granalie nach einem beliebigen vorangehenden Anspruch, wobei die Wachse ausgewählt sind aus der Gruppe bestehend aus PEG, ethoxylierten Fettalkoholen, Fettsäuren, Fettsäurealkoholen und Glyceriden.

11. Granalie nach einem beliebigen vorangehenden Anspruch, wobei die Granalien eine Klumpenbildung (caking strength) von weniger als 1000 haben.

12. Verfahren zum Herstellen einer Granalie nach Ansprüchen 1-11, umfassend das In-Kontakt-Bringen eines Partikels, umfassend eine aktive Verbindung, mit einer Umhüllung, wobei die Umhüllung eine Wachszusammensetzung mit einer Molekulargewichtsverteilung in dem Bereich umfasst von:

   (a) mindestens 10% (w/w) in dem Bereich 0,25xMw bis 0,75xMw, und
   (b) mindestens 20% (w/w) in dem Bereich 0,75xMw bis 1,25xMw, und
   (c) mindestens 10% (w/w) in dem Bereich 1,25xMw bis 2,0xMw,

   wobei Mw das Durchschnittsgewicht des Molekulargewichts der Wachszusammensetzung ist.

13. Verfahren nach Anspruch 12, wobei das In-Kontakt-Bringen des Partikels mit einer Umhüllung in einer Beschichtungskammer stattfindet.

14. Verfahren nach Anspruch 12, wobei das In-Kontakt-Bringen des Partikels mit einer Umhüllung in einer Flüssigbettapparatur oder in einer Mischapparatur stattfindet.

15. Futtermittel- oder Futterzusammensetzung für Tiere, umfassend die Granalie nach Ansprüchen 1 bis 11.

16. Verfahren zum Herstellen der Futtermittel- oder Futterzusammensetzung nach Anspruch 15, umfassend das Mischen einer Granalie nach Ansprüchen 1-11 mit einer Futtermittel- oder Futterzusammensetzung.

17. Teigzusammensetzung, umfassend die Granalie nach Ansprüchen 1 bis 11.

18. Verfahren zum Verbessern einer Teigzusammensetzung, umfassend das Mischen einer Teigzusammensetzung mit einer Granalie nach Ansprüchen 1-11.

19. Detergenszusammensetzung, umfassend eine Granalie nach Ansprüchen 1-11.

20. Verfahren zum Herstellen einer Detergenszusammensetzung, umfassend das Mischen einer Granalie nach Ansprüchen 1-11 mit einem oberflächenaktiven Mittel.

21. Düngerzusammensetzung, umfassend die Granalie nach Ansprüchen 1 bis 11.

22. Verfahren zum Herstellen einer Düngerzusammensetzung, umfassend das Mischen einer Granalie nach Ansprüchen 1-11 mit einem Düngemittel.

23. Pharmazeutische Zusammensetzung, umfassend die Granalie nach Ansprüchen 1 bis 11.


**Revendications**

1. Granule comprenant un noyau et un enrobage, le noyau comprenant une enzyme et l'enrobage comprenant une composition de cire ayant une répartition de poids moléculaire :

   (a) d'au moins 10% p/p dans la plage 0,25xPM à 0,75xPM, et
   (b) d'au moins 20% p/p dans la plage 0,75xPM à 1,25xPM, et
   (c) d'au moins 10% p/p dans la plage 1,25xPM à 2,0xPM, où PM est le poids moyen des poids moléculaires de la composition de cire.

2. Granule selon la revendication 1, où la température, $T_{m,i}$, à laquelle la composition de cire commence à fondre est au moins 25°C.

3. Granule selon l'une quelconque des revendications précédentes, où la $T_{m,i}$ de la composition de cire est au moins 30°C.

**4.** Granule selon l'une quelconque des revendications précédentes, où la $T_{m,i}$ de la composition de cire est au moins 35°C.

**5.** Granule selon l'une quelconque des revendications précédentes, où le point de fusion médian se situe entre 50 et 60°C.

**6.** Granule selon l'une quelconque des revendications précédentes, où le plage de fusion est au moins 10°C.

**7.** Granule selon l'une quelconque des revendications précédentes, où le PM est supérieur à 1000.

**8.** Granule selon l'une quelconque des revendications précédentes, où le PM est supérieur à 1200.

**9.** Granule selon l'une quelconque des revendications précédentes, où le PM est supérieur à 1400.

**10.** Granule selon l'une quelconque des revendications précédentes, où les cires sont choisies dans le groupe constitué de PEG, alcools gras éthoxylés, acides gras, alcools d'acides gras et glycérides.

**11.** Granule selon l'une quelconque des revendications précédentes, où les granules ont un pouvoir agglomérant de moins de 1000.

**12.** Méthode de préparation de granule selon les revendications 1 à 11, comprenant la mise en contact d'une particule comprenant un composé actif avec un enrobage, dans laquelle l'enrobage comprend une composition de cire ayant une répartition de poids moléculaire de l'ordre:

(a) d'au moins 10% p/p dans la plage 0,25xPM à 0,75xPM, et
(b) d'au moins 20% p/p dans la plage 0,75xPM à 1,25xPM, et
(c) d'au moins 10% p/p dans la plage 1,25xPM à 2,0xPM,

où PM est le poids moyen des poids moléculaires de la composition de cire.

**13.** Méthode selon la revendication 12, où ladite mise en contact de la particule avec un enrobage a lieu dans une chambre d'enrobage.

**14.** Méthode selon la revendication 12, où ladite mise en contact de la particule avec un enrobage a lieu dans un appareil sur lit fluidisé ou dans un appareil mélangeur.

**15.** Composition alimentaire ou de fourrage pour animaux comprenant la granule selon les revendications 1 à 11.

**16.** Méthode de préparation de la composition alimentaire ou de fourrage selon la revendication 15, comprenant le mélange d'une granule selon les revendications 1 à 11 avec une composition alimentaire ou de fourrage.

**17.** Composition pâteuse comprenant la granule selon les revendications 1 à 11.

**18.** Méthode pour améliorer une composition pâteuse comprenant le mélange d'une composition pâteuse avec une granule selon les revendications 1 à 11.

**19.** Composition de détergent comprenant une granule selon les revendications 1 à 11.

**20.** Méthode de préparation d'une composition de détergent comprenant le mélange d'une granule selon les revendications 1 à 11 avec un tensioactif.

**21.** Composition d'engrais comprenant la granule selon les revendications 1 à 11.

**22.** Méthode de préparation d'une composition d'engrais comprenant le mélange d'une granule selon les revendications 1 à 11 avec un agent fertilisant.

**23.** Composition pharmaceutique comprenant la granule selon les revendications 1 à 11.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8908694 A
- WO 9616151 A
- US 5480577 A
- EP 256127 A
- EP 304331 B1
- WO 9621716 A
- WO 9641859 A
- WO 9932595 A
- US 4106991 E
- US 4106991 A
- EP 170360 A
- EP 304332 A
- EP 304331 A
- EP 458849 A
- EP 458845 A
- WO 9739116 A
- WO 9212645 A
- WO 8908695 A
- WO 8707292 A
- WO 9106638 A
- WO 9213030 A
- WO 9307260 A
- WO 9307263 A
- WO 9638527 A
- WO 9723606 A
- WO 0123513 A
- US 5324649 A
- US 4689297 A
- EP 206417 A
- EP 193829 A
- DE 4344215
- DE 4322229 A
- DD 263790
- JP 61162185 A
- JP 58179492 B
- WO 2004003188 A
- WO 0228991 A
- EP 251446 A
- WO 9100345 A
- EP 525610 A
- WO 9402618 A
- WO 9606931 A
- DK 200200473
- DK 200101930
- WO 270608 B1
- WO 0001793 A
- WO 0125412 A
- WO 0220746 A
- WO 0228369 A

- US 5879920 A
- US 6348442 B
- DE 263790
- DK 0200885
- US 6136772 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- GB 1296839 A
- WO 9402597 A
- WO 9418314 A
- WO 9623873 A
- WO 9743424 A
- US 4435307 A
- US 5648263 A
- US 5691178 A
- US 5776757 A
- WO 8909259 A
- EP 0495257 A
- EP 0531372 A
- WO 9611262 A
- WO 9629397 A

- WO 9808940 A
- WO 9407998 A
- EP 0531315 A
- US 5457046 A
- US 5686593 A
- US 5763254 A
- WO 9524471 A
- WO 9812307 A

- DK 9800299 W
- WO 9324618 A
- WO 9510602 A
- WO 9815257 A
- WO 9219709 A
- WO 9219708 A
- US 4109991 A

**Non-patent literature cited in the description**

- **Orica, Sallay, P. et al.** Molar Mass Distribution of Hydroxyethylated Aralkyl Alcohols. *Periodica Polytechnica Ser. Chem. Eng.,* 2000, vol. 44 (2), 95-110
- **C.M. McTaggart.** *Int. J. Pharm.,* 1984, vol. 19, 139

- **Flanders.** *Drug Dev. Ind. Pharm.,* 1987, vol. 13, 1001
- Recommendations (1992) of the Nomenclature Committee of the International Union of Biochemistry and Molecular Biology. Academic Press, Inc, 1992
- **Dartois et al.** *Biochemica et Biophysica Acta,* 1993, vol. 1131, 253-360